# FASCICULE DE BREVET EUROPEEN

(11) **EP 0 383 681 B1**
(45) Date de publication et mention de la délivrance du brevet: **11.08.1993**
(21) Numéro de dépôt: 90400399.3
(22) Date de dépôt: 14.02.1990
(51) Int. Cl.: A61N 1/04, H01R 9/22

(54) **Bloc de contact à noyer dans un moulage électroconducteur destiné à un traitement électrique de la peau à but thérapeutique ou esthétique**
Kontaktvorrichtung für eine elektrisch leitende, zur therapeutischen oder ästetischen elektrischen Behandlung der Haut vorgesehene Auflage
Contact block to be countersunk in an electroconducting casting provided for a therapeutic or aesthetic electrical treatment of the skin

(30) Priorité: 17.02.1989 FR 8902102
(43) Date de publication de la demande: 22.08.1990
(73) Titulaire: Ramond, Gérard, F-31380 Montastruc-La-Conseillere (FR)
(72) Inventeur: Ramond, Gérard, F-31380 Montastruc-La-Conseillere (FR)
(74) Mandataire: CABINET BONNET-THIRION

(56) Documents cités:
- EP-A- 0 002 059
- EP-A- 0 004 514
- FR-A- 1 340 484
- US-A- 3 606 881

## Description

L'invention a trait à un bloc de contact à noyer dans la masse d'un moulage électroconducteur appliqué sur la peau d'un sujet en vue d'y faire passer un courant électrique, à but thérapeutique ou esthétique, fourni par un générateur de tension approprié, le bloc assurant la liaison électrique entre un câble relié au générateur et la masse du moulage électroconducteur.

Le document EP-A-0 004 514 décrit une électrode cutanée ou moulage électroconducteur, pour des traitements de la peau du corps humain, que l'on prépare sous forme d'un liant plastique avec un additif conducteur d'électricité; le liant est constitué d'une poudre susceptible de faire prise avec l'eau, typiquement plâtre de catégorie dentaire, et d'eau de prise en excès, et l'additif est un sel dissous dans l'eau ajoutée pour la prise, typiquement chlorure de calcium. Selon ce document EP-A-0 004 514, au moins un contact métallique est incorporé par enrobage dans le moulage.

La résistivité électrique du moulage électroconducteur est nettement plus élevée que celle d'un métal; on doit rechercher une résistivité surfacique analogue à celle que présente la peau, pour que la peau subisse des densités de courant comparables sous toute la surface du moulage électroconducteur, ce qui implique que les chutes de tension, entre le point où est injecté le courant et un point quelconque de la peau, soient sensiblement les mêmes dans le moulage et dans les tissus cutanés sous-jacents.

Toutefois, on a constaté que, sous les zones où sont incorporés les contacts métalliques, les densités de courant à l'entrée dans les tissus cutanés étaient supérieures aux densités de courant moyennes. En effet, du fait de leurs résistivités faibles, les contacts métalliques sont sensiblement équipotentiels, et les lignes de courant à l'entrée dans la matière du moulage sont normales à la surface du contact. Et, dans cette direction, la distance entre le contact et la peau dans le moulage est réduite, de sorte que le courant est concentré. Cela limite le courant efficace de traitement, et il y a des risques de brûlure de la peau si l'épaisseur de moulage est insuffisante sous le contact métallique; la mise en place du conducteur métallique enrobé dans le moulage demande du soin et de la compétence, et, même alors, il convient de doser prudemment l'intensité du courant.

L'invention vise à s'affranchir de ces inconvénients, et propose à cet effet un bloc de contact à noyer dans la masse d'un moulage électroconducteur appliqué sur la peau d'un sujet, en vue d'y faire passer un courant électrique à but thérapeutique ou esthétique fourni par un générateur de tension approprié, le bloc assurant la liaison électrique entre un câble relié au générateur et la masse du moulage électroconducteur, caractérisé en ce que le bloc comporte une plaquette souple en matériau conducteur avec deux faces sensiblement parallèles réunies par une tranche périphérique, et une garniture isolante surmoulée sur une première face de la plaquette et s'étendant autour de la tranche pour venir à fleur de la seconde face, découverte, une extrémité dénudée du câble de liaison s'encastrant dans la plaquette tandis qu'une gaine recouvre ce câble au moins sur la traversée du moulage.

Par cette disposition, l'injection de courant dans le moulage électroconducteur depuis le câble de liaison au générateur se fait uniquement par la deuxième face de la plaquette, la garniture isolante s'opposant au passage de courant à partir de la première face et de la tranche périphérique. On peut donc, dans une certaine mesure, choisir le trajet du courant vers la masse du moulage électroconducteur, pour éviter une concentration du courant dans une zone réduite de peau.

Accessoirement, la forme plate du bloc de contact est favorable à l'enrobage de ce bloc dans le moulage, pour minimiser les risques de déplacements en début de prise.

On préfère que la gaine du câble se termine dans la garniture avec laquelle elle fusionne, pour minimiser les risques de fuites électriques de l'âme du câble au moulage électroconducteur et les risques d'arrachement du câble.

Le matériau conducteur est de préférence un élastomère chargé de poudre conductrice; on allie ainsi aisément la souplesse de la plaquette et sa conductivité.

En disposition préférée, dans un moulage électroconducteur constitué avec une poudre susceptible de faire prise avec l'eau et un sel minéral en solution ionisée dans l'eau de prise, le bloc de contact reposera par sa garniture sur une première couche de moulage en contact avec la peau, tandis qu'une seconde couche, en continuité électrique avec la première, recouvre la seconde face de la plaquette.

On comprendra que la structure du bloc de contact est conçue et réalisée en vue de la disposition qui vient d'être présentée, avec la garniture qui interdit le passage direct de courant à travers la couche de moulage prise entre le bloc et la peau.

Mais le bloc de contact forme en soi un tout achevé, indépendant jusqu'au moment de l'exécution du moulage, et sa fonction propre resterait inchangée si la seconde face de la plaquette, découverte, était orientée vers la peau du sujet, encore que le résultat technique recherché (suppression des risques de concentration de courant) ne serait pas accompli.

Aussi semble-t-il nécessaire de définir l'invention sous l'aspect du bloc de contact en soi aussi bien qu'en association fonctionnelle orientée dans le moulage électroconducteur.

Des caractéristiques secondaires et les avantages de l'invention ressortiront d'ailleurs de la description qui va suivre, en référence aux dessins annexés dans lesquels:
- la figure 1 représente un bloc de contact selon l'invention, vu en plan;
- la figure 2 représente le bloc de contact de la figure 1, en élévation partiellement coupée;
- la figure 3 représente un bloc de contact selon l'invention, noyé dans un moulage électroconducteur formé sur la peau d'un sujet.

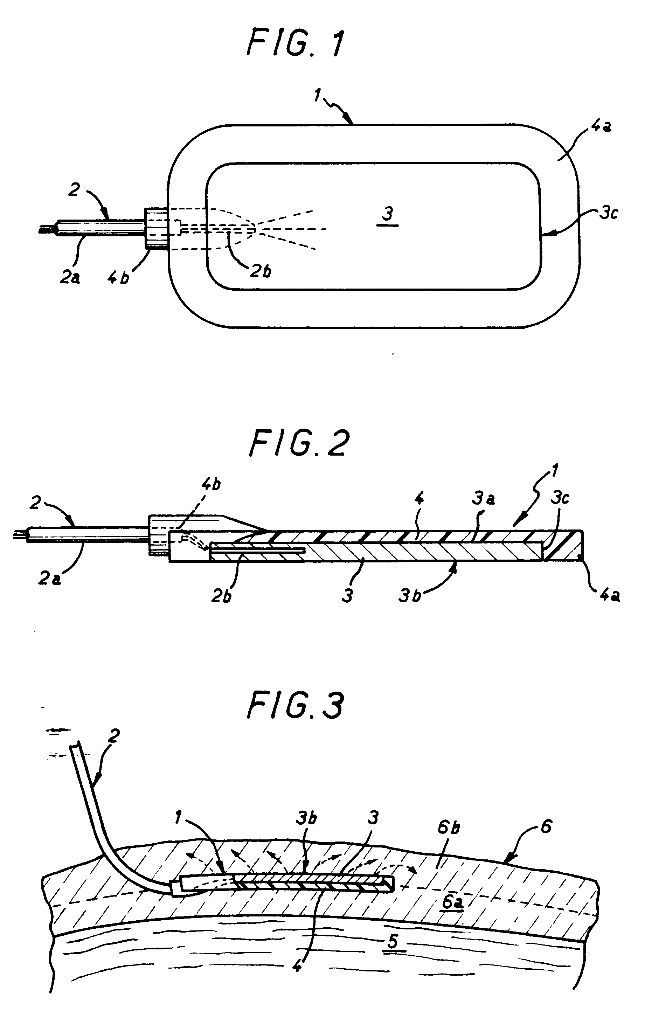

Selon la forme de réalisation choisie et représentée aux figures 1 et 2, un bloc de contact, 1 dans son ensemble, et destiné à établir un contact électrique entre l'âme 2b d'un câble 2 et un moulage électroconducteur, comme il sera précisé plus loin en référence à la figure 3, comprend une plaquette 3, sensiblement rectangulaire et d'épaisseur constante, réalisée en un élastomère, caoutchouc butyle ou silicone chargé d'une poudre conductrice, noir de carbone, poudre métallique, présentant une résistivité comprise entre 0,05 et 0,5 ohm.mètre.

La plaquette est délimitée par une première face 3a, une seconde face 3b, et une tranche périphérique 3c. Au moulage de la plaquette 3 on y a encastré l'extrémité dénudée de l'âme 2b du câble 2.

Sur la plaquette 3 est surmoulée une garniture isolante 4 qui recouvre la première face 3a de la plaquette 3, et s'étend, par un rebord 4a autour de la tranche périphérique 3c de la plaquette 3, jusqu'à venir à fleur de la seconde face 3b de cette plaquette.

Pour la sortie du câble 2, la garniture 4 comporte un appendice 4b, en saillie cylindrique du rebord 4a coaxiale avec le câble, dont la gaine 2a est fusionnée avec l'appendice 4b. Cet appendice 4b se termine en ogive sur la surface générale de la garniture.

Cette garniture peut être exécutée en polychlorure de vinyle, polyamide, en silicone et doit présenter une résistivité supérieure à 10⁶ ohm.mètre.

Comme représenté figure 3, pour traiter électriquement la peau d'un sujet 5, on gâche un mélange de plâtre dentaire avec 50% en poids d'eau contenant 4% en poids de chlorure de calcium et 2,5% de glycérol, et des adjuvants, selon les enseignements de EP-A-0 004 514.

Sur la peau du sujet 5, on applique une première couche 6a, d'épaisseur régulière, du mélange gâché; on applique sur cette première couche 6a un bloc de contact 1 avec sa garniture 4 plaquée à cette première couche 6a et la seconde face 3b de sa plaquette conductrice 3 tournée vers l'extérieur.

Puis, dès que la première couche 6a commence à prendre consistance, on complète le moulage 6 par une seconde couche 6b, qui recouvre le bloc de contact 1 en portant sur la seconde face 3b de la plaquette 3, et qui vient se reprendre sur la première couche 6a autour du bloc 1 pour être en continuité électrique avec cette première couche. Le câble de liaison 2 traverse la couche 6b pour émerger vers l'extérieur et se raccorder au câble.

On comprend aisément que la partie de la couche 6a qui se trouve entre la peau du sujet 5 et le bloc de contact, offrirait un passage, fortement conducteur par rapport à la masse du moulage pour le passage du courant, si la garniture 3 n'allongeait le cheminement des charges électriques depuis la face 3c de la plaquette 3 jusqu'à la peau.

Il va de soi que, pour des moulages qui intéressent une surface importante du corps du sujet notamment, on pourra utiliser plusieurs blocs de contact en parallèle. Par ailleurs, le générateur de tension électrique présente deux bornes; chacune de ces bornes peut être reliée à un ou plusieurs blocs de contact, suivant la structure de moulage utilisée.

Bien entendu, l'invention n'est pas limitée aux exemples décrits, mais en embrasse toutes ses variantes d'exécution.

## Revendications

1. Bloc de contact à noyer dans la masse d'un moulage électroconducteur (6) appliqué sur la peau d'un sujet (5), en vue d'y faire passer un courant électrique à but thérapeutique ou esthétique fourni par un générateur de tension approprié, le bloc (1) assurant la liaison électrique entre un câble (2) relié au générateur et la masse du moulage (6) électroconducteur, caractérisé en ce que le bloc (1) comporte une plaquette (3) souple en matériau conducteur avec deux faces (3a, 3b) sensiblement parallèles réunies par une tranche (3c) périphérique, et une garniture isolante (4) surmoulée sur une première face (3a) de la plaquette (3) et s'étendant autour de la tranche (3c) pour venir à fleur de la seconde face (3b), découverte, une extrémité dénudée (2b) du câble de liaison (2) s'encastrant dans la plaquette (3) tandis qu'une gaine (2a) recouvre ce câble au moins sur la traversée du moulage (6).

2. Bloc de contact selon la revendication 1, caractérisé en ce que la gaine (2a) du câble (2) se termine dans la garniture (4b) avec laquelle elle fusionne.

3. Bloc de contact selon l'une des revendications 1 et 2, caractérisé en ce que le matériau conducteur est un élastomère chargé d'une poudre conductrice.

4. Bloc de contact selon l'une quelconque des revendications 1 à 3, noyé dans un moulage électroconducteur (6) conformé sur la peau d'un sujet (5) pour y faire passer un courant électrique approprié, à partir d'une poudre susceptible de faire prise avec de l'eau, et d'eau de prise qui contient un sel minéral ionisé en solution, caractérisé en ce qu'il repose par sa garniture isolante (4) sur une première couche (6a) de moulage en contact avec la peau du sujet (5), tandis qu'une seconde couche (6b) de moulage en continuité électrique avec la première (6a) recouvre la seconde face (3b) découverte, de la plaquette (3).

## Claims

1. A contact block to be embedded in the body of an electrically conductive moulding (6) applied to the skin of a subject (5) for passing thereto an electric current for therapeutic or aesthetic purposes which is supplied by a suitable voltage generator, the block (1) providing an electrical connection between a cable (2) connected to the generator and the body of the electrically conductive moulding (6), characterised in that the block (1) comprises a flexible plate (3) of conductive material with two substantially parallel faces (3a, 3b) which are joined by a peripheral edge (3c), and an insulating lining (4) which is moulded on to a first face (3a) of the plate (3) and which extends around the edge (3c) to terminate flush with the second exposed face (3b), a bared end (2b) of the connecting cable (2) being inserted into the plate (3) while a sheath (2a) covers said cable at least where it passes through the moulding (6).

2. A contact block according to claim 1 characterised in that the sheath (2a) of the cable (2) terminates in the lining (4b) with which it is fused.

3. A contact block according to one of claims 1 and 2 characterised in that the conductive material is an elastcmer with a conductive powder filler.

4. A contact block according to any one of claims 1 to 3 embedded in an electrically conductive moulding (6) shaped to the skin of a subject (5) to pass thereto a suitable electric current, from a powder capable of setting with water, and setting water which contains an ionised mineral salt in solution, characterised in that it rests by way of its insulating lining (4) on a first moulding layer (6a) in contact with the skin of the subject (5) while a second moulding layer (6b) which is in electrical continuity with the first layer (6a) covers the second exposed face (3b) of the plate (3).

## Patentansprüche

1. Kontaktblock, der in die Masse eines elektrisch leitenden Gußkörpers (6) eingegossen ist, der auf die Haut eines Patienten (5) aufgelegt ist, um einen von einem geeigneten Spannungsgenerator gelieferten elektrischen Strom zu therapeutischen oder ästhetischen Zwecken hindurchfließen zu lassen, wobei der Block (1) die elektrische Verbindung zwischen einem mit dem Generator verbundenen Kabel (2) und der Masse des elektrisch leitenden Gußkörpers (6) gewährleistet, dadurch gekennzeichnet, daß der Block (1) eine biegsame Platte (3) aus leitendem Werkstoff mit zwei im wesentlichen parallelen Seiten (3a, 3b), die durch eine Umfangskante (3c) miteinander verbunden sind, und einen isolierenden Belag (4) umfaßt, der auf eine erste Seite (3a) der Platte (3) aufgegossen ist und sich um die Kante (3c) erstreckt, um mit der zweiten unbedeckten Seite (3b) bündig zu werden, wobei ein abisoliertes Ende (2b) des Verbindungskabels (2) in die Platte (3) eingelassen ist, während ein Mantel (2a) dieses Kabel wenigstens auf der Durchführung durch den Gußkörper (6) bedeckt.

2. Kontaktblock nach Anspruch 1, dadurch gekennzeichnet, daß der Mantel (2a) des Kabels (2) in dem Belag (4b) endet, mit dem es verschmilzt.

3. Kontaktblock nach einem der Ansprüche 1 und 2, dadurch gekennzeichnet, daß der leitende Werkstoff ein Elastomer ist, dem ein leitendes Pulver beigemischt ist.

4. Kontaktblock nach einem der Ansprüche 1 bis 3, der in einen elektrisch leitenden Gußkörper (6) eingegossen ist, der auf der Haut eines Patienten (5) zur Durchleitung eines geeigneten elektrischen Stroms ausgehend von einem Pulver, das mit Wasser abbinden kann, und Abbindewasser geformt ist, das ein ionisiertes mineralisches Salz in Lösung enthält, dadurch gekennzeichnet, daß er mit seinem isolierenden Belag (4) auf einer ersten Gußschicht (6a) aufliegt, die mit der Haut des Patienten (5) in Kontakt ist, während eine zweite Gußschicht (6b), die mit der ersten (6a) in elektrischer Kontinuität ist, die zweite unbedeckte Seite (3b) der Platte (3) abdeckt.
